# EUROPÄISCHE PATENTSCHRIFT

(11) **EP 1 725 202 B1**
(45) Veröffentlichungstag und Bekanntmachung des Hinweises auf die Patenterteilung: **12.09.2007**
(21) Anmeldenummer: 05726403.8
(22) Anmeldetag: 18.02.2005
(51) Int. Cl.: A61F 13/62, A61F 13/60, A44B 18/00

(54) **VERSCHLUSSBAND FÜR EINEN HYGIENEARTIKEL, HYGIENEARTIKEL UND VERFAHREN ZUM HERSTELLEN EINES VERSCHLUSSBANDES FÜR EINEN HYGIENEARTIKEL**
CLOSURE TAPE FOR A HYGIENE PRODUCT, HYGIENE PRODUCT, AND METHOD FOR THE PRODUCTION OF A CLOSURE TAPE FOR A HYGIENE PRODUCT
BANDE DE FERMETURE POUR ARTICLE D'HYGIENE, ARTICLE D'HYGIENE ET PROCEDE DE PRODUCTION D'UNE BANDE DE FERMETURE POUR ARTICLE D'HYGIENE

(30) Priorität: 18.02.2004 DE 102004008283
(43) Veröffentlichungstag der Anmeldung: 29.11.2006
(73) Patentinhaber: Koester GmbH & Co. KG, 96146 Altendorf (DE)
(72) Erfinder: NEUGEBAUER, Robert, 91077 Neunkirchen (DE); FELKL, Christian, 96149 Breitengüssbach (DE)
(74) Vertreter: Reuther, Martin
(86) Internationale Anmeldenummer: PCT/DE2005/000276
(87) Internationale Veröffentlichungsnummer: WO 2005/077314

(56) Entgegenhaltungen:
- EP-A- 0 983 760
- DE-U1- 8 327 230
- US-B1- 6 210 389

## Beschreibung

Die Erfindung betrifft ein Verschlussband für einen Hygieneartikel, einen Hygieneartikel und ein Verfahren zum Herstellen eines Verschlussbandes für einen Hygieneartikel (siehe US-A-6210389). Insbesondere betrifft die Erfindung ein Verschlussband für eine Babywindel oder für eine Inkontinenzwindel, eine Babywindel oder eine Inkontinenzwindel und ein Verfahren zum Herstellen solcher Windeln.

Beim Öffnen des Verschlussbandes einer Windel, um diese anzulegen oder um diese abzulegen, stellt sich regelmäßig die Aufgabe, das Verschlussband mit den Fingern an seinem Ende zu greifen und von der mit dem Band zusammenwirkenden Oberfläche der Windel oder eines Releasetapes an der Oberfläche der Windel abzuziehen. Hierzu hat ein Verschlussband oft einen Überstandsabschnitt, welcher als "freies Ende" über den Schließbereich eines solchen Bands hinausragt. Als besonders günstig herzustellen wird in der EP 0 840 585 B1 für Verschlussbänder mit einem Schließbereich und einem Überstandsabschnitt vorgeschlagen, in der Produktion zum Trennen von Bandstreifen einen wellenförmigen Schnitt längs der Maschinentransportrichtung vorzunehmen, wobei am wellenförmigen Bandende an jedem Wellenkopf ein Überstandsabschnitt entsteht.

Insbesondere vor einer Erstbenutzung liegen die Windelverschlussbänder herstellungsbedingt sehr fest auf der Windel oder auf einer ähnlichen Oberfläche auf. Mitunter schließen die Windeln, insbesondere im Falle von Inkontinenzwindeln, auch relativ fest. Von den mit den Fingern ergriffenen Überstandsabschnitten können die Fingerspitzen dann leicht abrutschen. Um dies zu vermeiden, müssen die Finger relativ fest am Überstandsabschnitt zudrücken, um das Windelverschlussband zuverlässig greifen bzw. die Windel zuverlässig öffnen zu können.

Um die Greifsicherheit des Überstandsabschnitts zu verbessern, wurden bislang zahlreiche Ansätze unternommen. Beispielsweise schlägt die japanische Patentanmeldung JP 62-142825 (Patentnummer JP 8-2365) vor, dass ein Rand des Überstandsabschnitts umgeklappt ist. Zusätzlich oder alternativ kann dort eine kleine Klebespur vorgesehen sein. In ähnlichen Ansätzen wurden auch durch Düsen aufgebrachte Heißsiegelklebeflächen am Überstandsabschnitt vorgeschlagen.

Andere Ansätze sehen vor, ein Hakenmaterial des Schließbereichs auch zum Rand des Verschlussbands hin aufzubringen. So sieht die US 6,210,389 eine durchgehende Hakenfläche vom Schließbereich bis zum Bandende vor, deren Haken zum Rand hin thermisch und mechanisch flachgebügelt werden oder zum Rand hin flachgeschnitten werden, um eine sicherere haptische Rückkopplung beim Greifen zu ermöglichen. Die US 5,624,429 schlägt demgegenüber vor, auf dem Überstandsabschnitt die gleichen oder ähnliche Haken wie im Schließbereich oder deren abgeschnittene Stümpfe anzuordnen, wobei im Überstandsbereich eine geringere Flächendichte der Haken vorgesehen sein soll. In beiden Fällen sind die jeweiligen Haken auf einer einzigen Trägerfläche bzw. -folie angeordnet.

Der vorliegenden Erfindung liegt die Aufgabe zu Grunde, ein Verschlussband bereitzustellen, welches mit einem sehr guten Kosten-Nutzen-Verhältnis eine hervorragende Rutschhemmung gegen das Abgleiten eines Fingers vom Windelverschlussband auch unter widrigen Einsatzbedingungen bietet und außerdem in der Herstellung flexibel an Kundenwünsche angepasst werden kann.

Diese Aufgabe löst die Erfindung gemäß der Ansprüche 1, 3, 4 und 12-14.

Begrifflich sei erläutert, dass der Schließbereich insbesondere eine Vielzahl von Haken aufweisen kann, beispielsweise über 100 Haken. Ein mit Haken versehener Schließbereich kann vorteilhaft als eine Komponente eines zwei Komponenten umfassenden Verschlusssystems, insbesondere eines Haken-Schlaufen-Verschlusssystems, fungieren. Geeignet in diesem Sinne sind sämtliche zusammenwirkfähigen Oberflächen, bei welchen aus einer Oberfläche hervorstehende Anordnungen der einen Komponente hinter Aussparungen, Schlaufen, Öffnungen, Fasern oder ähnliches der anderen Komponente greifen können, sodass sich gegen ein Ablösen der beiden Oberflächen voneinander eine Widerstandskraft ergibt. Die hervorstehenden Anordnungen der einen Komponente können neben Haken zahlreicher Formen auch pilzkopfförmige Stäbchen oder andere geeignete Formen sein.

Der Befestigungsbereich des Windelverschlussbands dient dazu, dieses permanent an einer Oberfläche zu befestigen, beispielsweise am Windelohr einer Windel. Somit ergibt sich am gegenüberliegenden Ende ein freies Ende des Bandes, das sogenannte Benutzerende. In dessen Nähe ist der Schließbereich angeordnet, welcher dazu dient, zum Verschließen der Windel nach dem Anlegen vom Benutzer auf eine mit dem Schließbereich zusammenwirkende Fläche meist am gegenüberliegenden Windelohr bzw. auf der Bauchseite der angelegten Windel geführt zu werden und die Windel somit öffenbar zu verschließen. Hierzu kann das Windelverschlussband insbesondere am Überstandsabschnitt gegriffen werden, also demjenigen Abschnitt, welcher am Benutzerende des Bandes auf der unbefestigten Seite des Schließbereichs liegt.

Ein Windelverschlussband erstreckt sich üblicherweise in einer Längserstreckung vom Befestigungsbereich zum Überstandsabschnitt. Oft ist ein Windelverschlussband rechteckig oder zumindest im Wesentlichen rechteckig geformt.

Vorteilhaft gegenüber dem Vorbekannten stellt der vorgestellte Aspekt der Erfindung einen erheblich verbesserten Kosten-Nutzen-Kompromiss dar: Gegenüber einem bis zum Rand des Verschlussbands durchgehenden Hakenmaterial oder gegenüber dessen bis zum Rand durchgehender Kunststoffbasis kann der Greifbereich auf der Maschine besonders leicht im Laufe der Produktion geändert werden. Der Greifbereich kann auf diese Weise schnell und kostengünstig an Kundenwünsche angepasst werden. Dieser Vorteil wird dadurch erreicht, dass der Greifbereich separat ausgestaltet ist, also von den benachbarten Baugruppen des Windelverschlussbandes, insbesondere beispielsweise von dem Schließbereich, getrennt ausgebildet ist bzw. materiell andere Baugruppen aufweist.

Den preisgünstigen Weg zur Abrutschsicherheit erreicht die Erfindung nach dem vorgestellten Aspekt dadurch, dass sie eine "makroskopisch strukturierte Oberfläche" welche eine Prägung aufweist, für den separaten Greifbereich vorschlägt. Der Greifbereich soll bevorzugt so am Band angeordnet sein, dass es sich über dessen Oberfläche deutlich erhebt, bevorzugt genau um seine eigene Dicke, also um die Dicke eines entsprechenden Bauelements. Der eigentliche Greifbereich befindet sich dann an der vom Band abgewandten Oberfläche des strukturell separaten Bauelements. An dieser Oberfläche soll eine makroskopische Struktur vorliegen, was insbesondere heißt, dass die Struktur mit einem Finger ertastbar ist. Vorteil einer solchen relativ großdimensionalen Struktur ist es unter anderem, dass die Finger auch bei widrigen Umständen sicher Halt am Überstandsabschnitt und somit am Benutzerende des Windelverschlussbands finden. Widrige Umstände können insbesondere dann vorliegen, wenn das Benutzerende des Verschlussbandes oder aber die Finger des Benutzers feucht, nass oder verschmutzt ist bzw. sind. Gerade beim Anlegen von Babywindeln kann dies oft nicht ausgeschlossen werden. Insofern ist die vorgeschlagene makroskopische Oberflächenstruktur insbesondere vorteilhaft gegenüber einem Klebestreifen, so wie er in der JP 8-2365 vorgeschlagen wird.

Gegenüber einer auflaminierten Folie oder einer Heißsiegelklebeschicht ergibt sich durch die makroskopisch strukturierte Oberfläche eine bessere Griffigkeit. Begrifflich sei hierzu erläutert, dass eine Folie an sich eine im Wesentliche zweidimensionale Erstreckung hat und zumindest auf ihrer Oberfläche im makroskopischen Maßstab völlig oder zumindest weitestgehend glatt ist. Demgegenüber verleiht eine punktuell oder flächig aufgebrachte oder ausgebildete Struktur die Grifffestigkeit an der Oberfläche.

Insofern bietet vorliegende Erfindung gegenüber den aus dem Stand der Technik bekannten, als Greifbereich ausgestalteten Hakenflächen der Schließbereiche den Vorteil, dass der Greifbereich nach vorliegender Erfindung einfach und kostengünstig für seine Aufgabe, eine gute Griffigkeit aufzuweisen, ausgestaltet werden kann. Schon die vergrößerte Hakenfläche aus dem Stand der Technik ist unverhältnismäßig kostspielig. Umso aufwändiger und kostspieliger ist dann noch eine Nachbearbeitung dieser Hakenfläche. Gegenüber den aus dem Stand der Technik bekannten, folienartigen Greifbereichen hat vorliegende Erfindung den Vorteil, dass durch die makroskopisch strukturierte Oberfläche eine wesentlich höhere Griffigkeit des Greifbereiches erzielt werden kann. Insbesondere kann die Griffigkeit verhältnismäßig kostengünstig an geänderte Randbedingungen bzw. an geänderte Kundenwünsche angepasst werden.

Insbesondere wenn der Greifbereich als vom eigentlichen Windelverschlussband eigenständiges strukturelles Bauteil aufgebracht ist, kann der Greifbereich schnell und kostengünstig an Kundenwünsche angepasst werden. Dadurch, dass der Greifbereich durch ein eigenständiges Bauteil erzeugt wird, können auch die haptischen und übrigen mechanischen Eigenschaften des Greifbereichs gezielt und unabhängig vom übrigen Windelverschlussband ohne Weiteres eingestellt, gewählt, angepasst und/oder geändert werden. Dieses kann insbesondere auch ohne Weiteres vor der Applikation an dem übrigen Windelverschlussband bzw. an einem Trägerband des Windelverschlussbands geschehen. Andererseits kann dieses selbstverständlich auch nach der Applikation erfolgen.

Darüber hinaus ist es auch denkbar, dass der separate Greifbereich durch ein strukturelles Bauelement des übrigen Windelverschlussbandes gebildet wird, wie beispielsweise durch eine Trägerfolie des Windelverschlussbandes. Hierbei ist zu beachten, dass jedoch die zum Überstandsabschnitt bzw. zum Greifbereich benachbarte funktionelle Einheit bzw. die zum Überstandsabschnitt bzw. zum Greifbereich benachbarten funktionellen Einheiten des Windelbverschlussbandes strukturell anders aufgebaut sind, damit nach wie vor der Greifbereich separat ausgebildet ist.

Bei einer derartigen Anordnung kann es insbesondere vorteilhaft sein, die makroskopische Struktur der Oberfläche welche eine Prägung aufweist im separaten Greifbereich erst nachträglich, insbesondere nach dem Zusammenbringen der wesentlichen Komponenten des Windelverschlussbandes, bereitzustellen.

Ein besonders kostengünstiges Bereitstellen, Auftragen bzw. Anbringen des Bauteils für den Greifbereich löst die Erfindung nach einer besonders bevorzugten Ausführungsform dadurch, dass der Greifbereich "folienartig" ausgebildet ist. Hierbei wird in vorliegendem Zusammenhang mit dem Begriff "folienartig" eine Baugruppe bezeichnet, deren Fläche in mm² zumindest den achtfachen Betrag der zu dieser Fläche gemessenen Stärke oder Dicke in mm aufweist. Hierbei handelt es sich dann in vorliegendem Fall bei einem derartigen Bauteil um eine Folie, wenn diese ausreichend eigenstabil und biegsam ist. Um einen folienartigen Greifbereich zu erhalten, kann beispielsweise ein Folienstück als separates Bauteil auf dem Überstandsabschnitt aufgeklebt oder sonst wie angebracht werden, was mit wenig Aufwand verbunden ist. Alternativ kann ein unmittelbar haftender Werkstoff, beispielsweise ein Heißsiegelklebstoff oder ein anderer thermoplastischer Werkstoff wie beispielsweise TPE, unmittelbar auf das Windelverschlussband so aufgetragen werden, dass sich wiederum ein am Windelverschlussband befindliches eigenständiges strukturelles Bauteil in der Art und der Beschaffenheit einer Folie ausbildet. Insbesondere im Fall von Heißsiegelklebstoffen kann das Bauteil jedoch auch verhältnismäßig spröde sein, so dass hier besser von einem folienartigen Bauteil gesprochen wird. Ein folienartiger Werkstoff für das strukturell eigenständige, den separaten Greifbereich bildende Bauelement ist insbesondere erheblich günstiger als ein mechanisch nachbearbeitetes Hakenmaterial, so wie die US 6,210,389 vorsieht, oder als das Vorsehen einiger einzeln angeordneter Haken oder Hakenflecken, so wie dies in der US 5,624,429 vorgeschlagen wird.

Damit der Greifbereich besonders gut ertastbar ist und sich auch von einer benachbart angeordneten Hakenfläche abheben kann, wird vorgeschlagen, dass er ein strukturell eigenes Bauelement in Form einer Folie mit einer Dicke von über 100 µm, bevorzugt allerdings von weniger als 600 µm, aufweist. Je nach der konkreten Art der makroskopischen Oberflächenstruktur müssen an die separate Folie des Greifbereichs keine großen mechanischen Anforderungen gestellt werden. Daher können auch relativ dicke Folien mit geringem Kostenaufwand eingesetzt werden. Selbst wenn technisch anspruchsvollere Folien verwendet werden, ist dennoch davon auszugehen, dass die vorgeschlagene Lösung recht preiswert bleibt. Insbesondere können Nachbehandlungsschritte - wie beispielsweise in der US 6,210,389 B1 erforderlich - entfallen.

Die strukturierte Oberfläche weist eine Prägung auf, die eine verbesserte haptische Eigenschaft verleiht. Eine Prägung bewirkt bei geringen Kosten eine spürbare Erhöhung der Rutschhemmung. Alternativ bzw. kumulativ zu der Prägung kann der Greifbereich auch einen Rand aufweisen, der zu einem Trägerband oder ähnlichem abfällt und in haptischer Hinsicht wie eine Prägung wirkt, so dass auf diese Weise eine makroskopische strukturierte Oberfläche gebildet wird. Insbesondere in letzterem Fall kann dann auf eine makroskopische Strukturierung der übrigen Oberfläche des Greifbereichs verzichtet werden.

Der Greifbereich kann bewusst isotrop oder anisotrop in seinen Dehneigenschaften und Biegeeigenschaften eingestellt werden, wenn die Prägungen eine Mehrzahl von geraden und gekrümmten Linien aufweisen, von denen vorzugsweise einige miteinander verbunden sind. Beim Greifen des Greifbereichs mit dem Finger können diese zudem sehr angenehm als Prägungshäufungen bevorzugte Anfassbereiche bieten. Die dergestalt gruppierten Linien können insbesondere Buchstaben und/oder Zahlen ergeben.

Der Greifbereich kann an einem Greifrand des Überstandsabschnitts angeordnet sein, wobei der Greifbereich beispielsweise streifenartig ausgebildet sein kann und in seiner Form zumindest teilweise im Wesentlichen dem Greifrand entsprechend verlaufen kann. Er kann beispielsweise geometrisch ähnlich zum Greifrand des Überstandsabschnitts verlaufen, an der Bandkante entlang verlaufen oder mit Abstand zur Bandkante angeordnet sein. Auch kann der Greifbereich kreisflächenförmig oder kreisringförmig sein. Anzahl, Größe und Form sind wenigen Einschränkungen unterworfen. Insbesondere können zahlreiche geometrische Grundformen oder deren Kombinationen sowie Beschriftungen oder graphische Darstellungen für die Form des Greifbereichs verwendet werden. Der Greifbereich kann zudem in mehreren Greifbereichsinseln vorliegen, die bevorzugt sämtlich innerhalb des Überstandsabschnitts angeordnet sind. Insbesondere wenn der Greifbereich am Rand des Überstandsabschnitts verläuft, aber nicht auf diesen Fall eingeschränkt, kann es von Vorteil sein, wenn der Greifbereich mäanderförmig verläuft.

Wenn der Greifbereich am Greifrand oder zumindest nahe zur Bandkante angeordnet ist, ist er für die Fingerspitzen in der Regel am besten zu ertasten. Gleiches gilt dann, wenn der Greifbereich geometrisch ähnlich oder zum Greifrand oder zumindest an dessen Form angelehnt ausgebildet ist. Bei einem Versatz zwischen Greifbereich und dem Bandkante hingegen ist der Greifbereich regelmäßig optisch besser erkennbar. Gleiches gilt dann, wenn der Greifbereich eine deutlich andere Geometrie hat als der Greifrand des Überstandsabschnitts.

Der Greifbereich ist sowohl haptisch als auch optisch besonders leicht zu finden, wenn er etwa einen gleichen Abstand zu dem Schließbereich wie zum dem Schließbereich zuzuordnenden Bandende aufweist.

Der separate folienartige Greifbereich mit der makroskopisch strukturierten Oberfläche kann sowohl auf einer Seite des Verschlussbands als auch auf beiden Seiten des Verschlussbands angeordnet sein. Wenn nur auf einer Seite des Verschlussbands ein solcher Greifbereich vorgesehen ist, kann dies auf derjenigen Seite des Bandes gegeben sein, auf welcher auch der Schließbereich angeordnet ist. In diesem Fall wird er bei einer üblichen Benutzung am ehesten dem Daumen einer Hand des Benutzers Halt geben. Wenn ein Greifbereich auf derjenigen Seite angeordnet ist, welche frei vom Schließbereich, also auf der gegenüberliegenden Seite eines den Schließbereich tragenden Trägerbandes angeordnet, ist, werden eher die gruppierten Finger der Hand des Benutzers auf ihnen zum Liegen kommen. Dementsprechend wird auch vorgeschlagen, dass sich der folienartige Greifbereich mit der makroskopisch strukturierten Oberfläche auf der den Schließbereich tragenden Bandseite vollständig neben diesem findet.

Alternativ oder kumulativ kann der separate Greifbereich auf der gegenüberliegenden Seite bevorzugt ebenfalls vollständig neben dem Schließbereich liegen; ein Teil des Greifbereichs auf dieser Seite oder eine zusätzliche Insel des Greifbereichs kann allerdings auch unterhalb des Schließbereichs vorliegen. Dies bietet insbesondere den gruppierten Fingern der Hand des Benutzers eine größere Angriffsfläche. Begrifflich sei erläutert, dass der Greifbereich vollständig neben dem Schließbereich im Sinne der vorliegenden Anmeldung angeordnet ist, wenn sich bei einer Projektion des Schließbereichs und des Greifbereichs auf eine zum Windelverschlussband parallele Ebene keine Überschneidung, bevorzugt sogar keine unmittelbare Angrenzung ergibt.

In einer bevorzugten Ausführungsform mit separaten folienartigen Greifbereichen auf beiden Seiten des Verschlussbands sind zumindest ein erster Greifbereich und ein zweiter Greifbereich auf verschiedenen Seiten des Bandes gleich groß und einander deckend angeordnet. Hierdurch fühlt sich der Überstandsabschnitt sehr stabil an. Eine solche Konstellation, aber auch die übrigen genannten Merkmale, können auch dann erreicht werden, wenn der Überstandsabschnitt einen separaten folienartigen Greifbereich mit einer makroskopisch strukturierten Oberfläche aufweist, wobei die separate Folie des Greifbereichs auf beiden Seiten des Verschlussbands angeordnet ist. Insbesondere kann sie hierzu um das Stirnende des Verschlussbands herumreichen.

Es versteht sich, dass die Anordnung eines Greifbereichs an der Außenseite des Windelverschlussbandes, insbesondere somit an der Seite, welche den Schließbereich nicht trägt, auch unabhängig von den übrigen Merkmalen vorliegender Erfindung vorteilhaft ist, um die Stabilität einer Rolle, die zum Herstellen des Windelverschlussbandes dann noch quer geschnitten wird, zu erhöhen. Hierbei kann vorzugsweise das Material des Greifbereichs derart gewählt werden, dass es sowohl eine gute Haptik gewährleistet als auch eine guten Halt einer auf dem Greifbereich liegenden zweiten Lage der Rolle gewährleistet. Hierbei sollte auch dieser Greifbereich eine makroskopisch strukturierte Oberfläche aufweisen und vorzugsweise folienartige bzw. als Folie ausgebildet sein.

Weiterhin sei begrifflich erläutert, dass der Überstandsabschnitt insbesondere der gesamte über die äußersten Schließmittel des Verschlussbands zum Rand des Verschlussbands hinausragende Abschnitt des Verschlussbands sein kann. Als dasjenige Bandende, welches dem Schließbereich zuzuordnen ist, wird das Bandende verstanden, welches näher am Schließbereich angeordnet ist, wohingegen einem gegenüberliegen Bandende der Befestigungsbereich des Verschlussbands näher liegt. Es sei darauf hingewiesen, dass unter dem Bandende das Stirnende des längs vom Befestigungsbereich zum Benutzerende erstreckten Verschlussbands verstanden werden soll. Das Windelverschlussband selbst ist hierbei eine untrennbare Struktur, die unter dem Schließbereich und dem Greifbereich hindurch verläuft.

Der Überstandsbereich ist demzufolge üblicherweise frei von den Schließmitteln. Bei einem Windelverschlussband nach der WO 97/32555 A1, bei welcher ein zusätzliches Schließmittel auf jedem Windelverschlussband vorgesehen ist, ist lediglich der schmale Streifen neben dem zusätzlichen Verschlussmittel als Überstandsbereich aufzufassen. Dies kann jedoch nicht verallgemeinert werden, denn selbstverständlich unterliegt auch ein Windelverschlussband mit einem ausgeprägten Schließbereich und nur einigen vereinzelten, funktionell praktisch untätigen Haken im eigentlichen Überstandsbereich dem Gedanken der vorliegenden Erfindung. Konkret unterliegt insofern also dem vorliegenden Erfindungsgedanken auch ein solches Windelverschlussband, bei welchem - im Verlauf vom Befestigungsbereich zum Benutzerende hin - ein Schließbereich, dann ein separater folienartiger Greifbereich mit einer makroskopisch strukturierten Oberfläche und abschließend einige ganz wenige, funktional gegenüber dem Schließbereich ganz erheblich zurücktretende, Haken angeordnet sind. Bevorzugt ist jedoch der Überstandsbereich vollständig frei von den mechanischen Mitteln des Schließbereichs.

Ebenfalls löst die Aufgabe ein Verfahren zum Herstellen eines Verschlussbands für einen Hygieneartikel der genannten Art, bei welchem Verfahren eine geprägte makroskopisch oberflächenstrukturierte Folie von einer Rolle oder einer Spule auf den Überstandsabschnitt des Abschlussbands laminiert wird. Als Alternative hierzu kann zunächst eine strukturell eigene Baugruppe, wie ein thermoplastisches Material, TPE und/oder ein Heißsiegelklebstoff, beispielsweise über eine Düse, eine Sprüheinrichtung, eine Walze oder mit anderen geeigneten Mitteln auf den Überstandsabschnitt aufgebracht werden. Kumulativ bzw. alternativ ist es möglich, einen Bereich des Bandes zur Bereitstellung eines Greifbereichs mit einer makroskopischen Oberflächenstruktur zu versehen, was beispielsweise mit einer Prägewalze und/oder einem Prägerad geschehen kann. Dies kann bevorzugt geschehen, bevor das aufgebrachte Material nach dem Aufbringen seine Formbarkeit verliert, um es nicht nochmals erhitzen zu müssen.

In diesem Zusammenhang sei erläuternd betont, dass Windelverschlussbänder in der Regel in ihrem strukturellen Aufbau auf Rollen bereitgestellt werden, wobei diese Rollen dann in einzelne Streifen geschnitten werden, welche dann erst als Windelverschlussbänder an einer Windel appliziert werden. Die in vorliegendem Zusammenhang aufgeführten Überlegungen hinsichtlich des Aufbaus der Windelverschlussbänder und deren Herstellungsverfahren betreffen gleichermaßen auch etwaige Rollen oder Spulen als Zwischenprodukte, aus denen die Windelverschlussbänder durch Trennvorgänge, wie beispielsweise Quer oder Längsschnitte, bereitgestellt werden, insoweit nicht an entsprechender Stelle dieses ausdrücklich verneint ist. Hierbei können einzelne Verfahrensschritte unmittelbar an der Windelmaschine oder zu anderen Zeitpunkten bzw. bei bereits vereinzelten Windelverschlussbändern, bei Spulen oder Rollen sowie bei Zwischenprodukten für die Herstellung dieser Spulen oder Rollen vorgenommen werden.

Die Erfindung wird anhand verschiedener Ausführungsbeispiele unter Bezugnahme auf die Zeichnung nachfolgend weiter erläutert. In der Zeichnung können funktional gleiche Bauelemente gleiche Bezugsziffern tragen. Es zeigen
- Figur 1: schematisch einen Querschnitt durch zwei aneinanderliegende Windelverschlussbandstreifen, wie sie auf einer Herstellungsmaschine transportiert und geschnitten werden,
- Figur 2: schematisch eine Schneidelinie, entlang welcher die Windelverschlussbandstreifen aus Figur 1 bei der Produktion getrennt werden,
- Figur 3: schematisch eine Draufsicht auf einen Windelverschlussbandstreifen nach Trennung vom gegenüberliegenden Streifen, jedoch vor Zerschneiden in die eigentliche Bandform,
- Figuren 4 und 5: jeweils zwei an einer Windel befestigte Windelverschlussbänder und
- Figuren 6a bis 6d: verschiedene Ausführungsbeispiele der Erfindung im Detail gemäß beispielhafter Kennzeichnung in Figur 3.

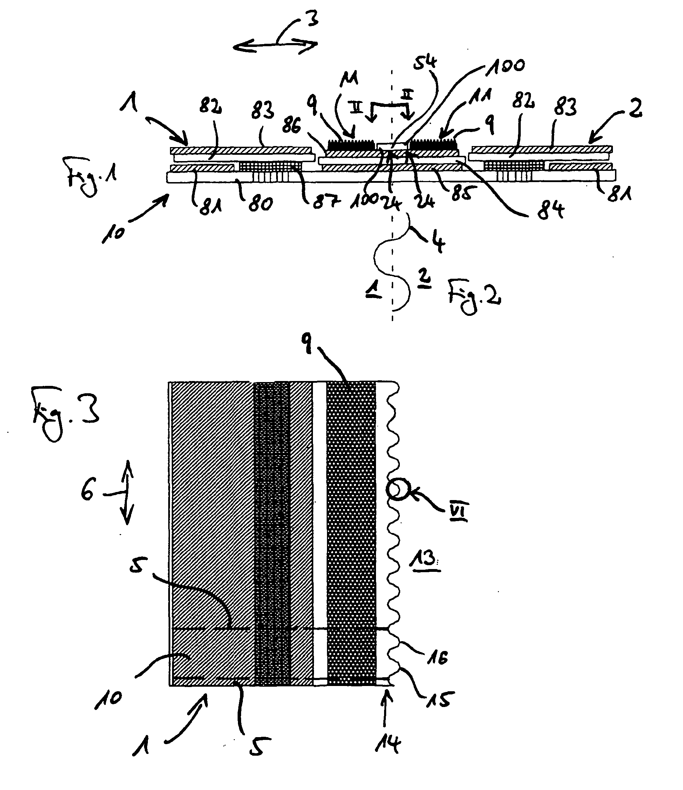

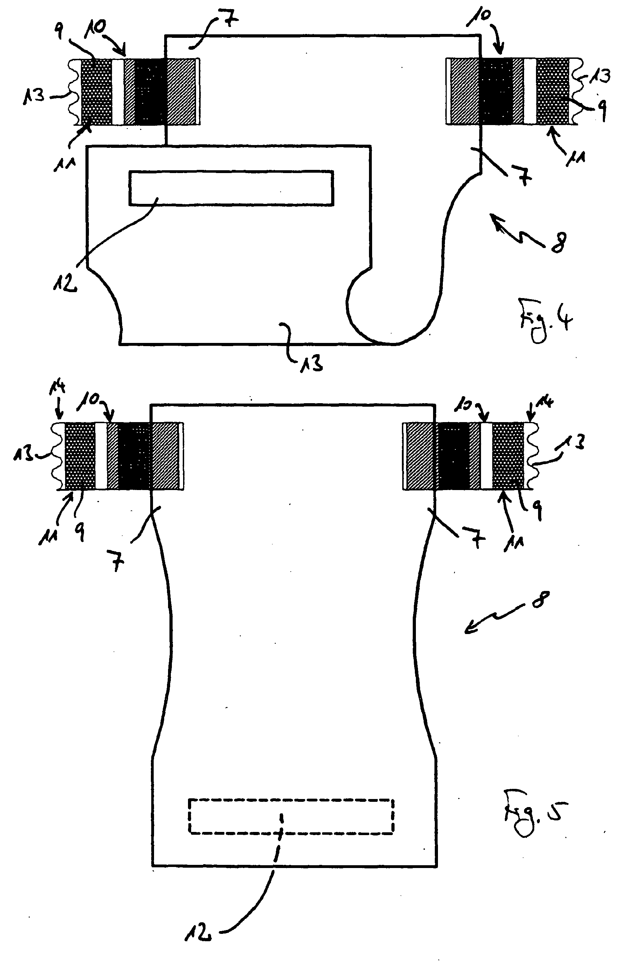

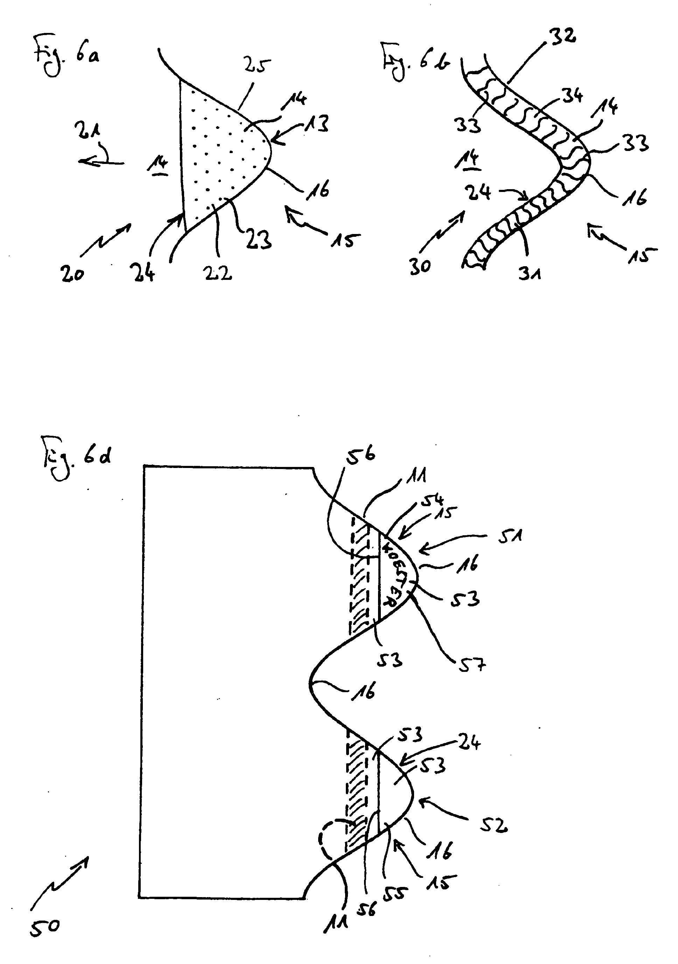

Die beiden Verschlussbandstreifen 1 und 2 in Figur 1 sind in einer machine cross direction 3 stirnseitig verbunden und laufen in dieser Form üblicherweise durch die Herstellungsmaschine, welche sie beispielsweise wellenförmig (vergleiche Figur 2) entlang eines Schnitts 4 trennt. Nach der Trennung durch den Schnitt 4 in Maschinenquerrichtung 3 wird der Windelverschlussbandstreifen 1 durch Trennschnitte 5 (exemplarisch gekennzeichnet in Figur 3) zu einzelnen Windelverschlussbändern 10 separiert, welche in Maschinenrichtung 6 zueinander benachbart liegend aus der Maschine entnommen werden können.

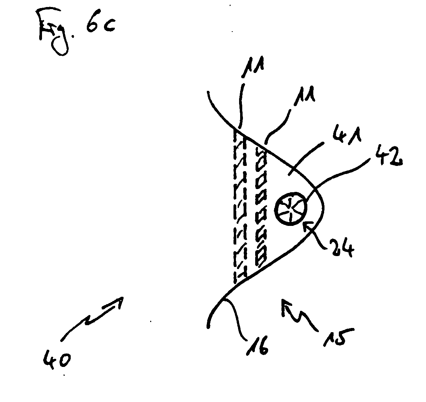

Die Windelverschlussbänder 10 werden an Windelohren 7 (exemplarisch gekennzeichnet in Figuren 4 und 5) der Windel 8 befestigt, sodass sie über Haken 9 eines Schließbereichs 11 die Windel 8 sicher öffenbar verschließen können. In einem Frontaltape 12 an einer Außenseite 13 der Windel sind hierzu bei diesen Ausführungsbeispielen mit den Haken 9 korrespondierende Schlaufen vorgesehen.

Zum Greifen eines Benutzerendes 13 des Windelverschlussbandes 10 ist am Benutzerende 13 ein Überstandsbereich 14 vorgesehen. Dieser grenzt unmittelbar an den mit Haken 9 versehenen Schließbereich 11 an und erstreckt sich von dort in wellenkopfförmige Laschen 15 des Windelverschlussbands 10 hinaus bis zu einer stirnseitigen Bandkante 16.

Neben den Schließbereichen 11 der jeweiligen Windelverschlussbänder 10 erheben sich vom übrigen Band strukturell unabhängige Bauelemente 100 auf den Überstandsabschnitten 14 und bilden so separate folienartige Greifbereiche 24. Diese verkörpern die Erfindung. In ihrer genauen Form und Anzahl sind die Bauelemente 100 wenigen Einschränkungen unterworfen.

Beispielhaft sind auf den Überstandsabschnitten der Ausführungsbeispiele in den Figuren 6a, 6b, 6c und 6d unterschiedliche separate folienartige Greifbereiche 24 bildende strukturell eigenständige Bauelemente 100, 22, 31, 42, 54, 55 mit einer makroskopisch strukturierten Oberfläche vorgesehen:

Die wellenkopfförmige Spitze des Überstandsabschnitts 14 des ersten Ausführungsbeispiels 20 in Figur 6a ist frei von Schließmitteln. Der Schließbereich des ausschnittsweise dargestellten Verschlussbands befindet sich in einer Richtung 21 zum Befestigungsende des Verschlussbandes hin versetzt außerhalb des dargestellten Bereichs. Auf den wellenkopfförmigen Bereich 15 des Überstandsabschnitts 14 ist eine etwa 400 µm dicke Folie 22 auflaminiert. Die Folie 22 weist zahlreiche makroskopische Perforationspunkte 23 auf, welche die ansonsten makroskopisch glatte Oberfläche der Folie 22 unterbrechen. Die Perforationspunkte 23 liegen gleichmäßig angeordnet in einem Gitternetz, welches gegenüber der Haupterstreckungsrichtung 21 des Windelverschlussbands um 45° gedreht angeordnet ist.

Die Perforationspunkte 23 versehen die Folie 22 mit einer makroskopischen Oberflächenstruktur, welche diesem Teil des Überstandsabschnitts 14 eine hohe Griffigkeit verleiht. Daher dient die oberflächenperforierte Folie 22 als separater Greifbereich 24. Der Greifbereich 24 erstreckt sich bis an die Bandkante 16, sodass sich ein griffiger Greifrand 25 an der Bandkante 16 ergibt.

Es sei darauf hingewiesen, dass die Vielzahl der Perforationen 23 auch ungeordnet oder in einem anderen Muster vorliegen kann. Den sinnvollen Möglichkeiten sind hier kaum Grenzen gesetzt. Beispielsweise könnten die Punkte in einem alternativen Muster auch als konzentrische Ringe, Dreiecke, Quadrate oder Rechtecke bildend oder beispielsweise in geometrisch ähnlichem Verlauf zur Bandkante 16 in die Folie eingebracht sein. Auch können statt Perforationspunkten 23 perforierte Sterne, Kreuze, Linien, Dreiecke, Quadrate, Ovale oder andere Formen gewählt werden.

Die zweite Ausführungsform 30 in Figur 6b (wobei der Ausschnitt entsprechend demjenigen in Figur 6a gewählt ist) trägt an der Bandkante 16 des wellenkopfförmigen Bereichs 15 des Überstandsabschnitts 14 eine in sich gewellt aufgebrachte Folie 31. Die Folie 31 ist ein etwa 5mm breiter Streifen, der unmittelbar entlang der Bandkante 16 über deren gesamte Länge verläuft. Somit ergibt sich auch hier ein griffiger Greifrand 32 am Windelverschlussband. Entlang des in der Fläche des Verschlussbands mäandrierenden Verlaufs des Folienstreifens 31 ist dieser aus der Bandebene heraus gewellt, sodass sich zahlreiche zu ihren Nachbarn im Wesentlichen parallele Kämme 33 (exemplarisch gekennzeichnet) so erheben, dass beim Greifen des Überstandsabschnitts 14 die Finger diese Kämme 33 als erstes berühren und gut greifen können. In Tälern 34 (exemplarisch gekennzeichnet) ist die streifenförmige Folie 31 am Windelverschlussband fixiert.

Das dritte Ausführungsbeispiel 40 in Figur 6c hat auf seiner wellenkopfförmigen Lasche 15 einen in zwei Streifen aufgeteilten Schließbereich 11, sodass sich ein verkleinerter Überstandsabschnitt 41 an der Spitze der Lasche 15 ergibt. Etwa zentriert im Überstandsabschnitt 41 ist auf das Verschlussband ein kreisförmiger Folienfleck 42 auflaminiert. Der Folienfleck 42 ist an seiner Oberfläche mit radial angeordneten Streifen eingeprägt. Die Folie 42 des Greifbereichs ist etwa 300 µm dick und auf der gegenüberliegenden Seite der Schließbereiche 11 angeordnet. Bei einer üblichen Anordnung eines solchen Windelverschlussbandes an einer herkömmlichen Windel dergestalt, dass der Schließbereich zur Innenseite der angelegten Windel hin gerichtet ist, kommt der Folienfleck 42 demzufolge außen an der Windel zum Liegen.

Auf der Innenseite, also auf derjenigen Seite des Verschlussbands, auf welcher auch die Schließbereiche 11 angeordnet sind, kann eine weitere separate Folie mit einer makroskopisch strukturierten Oberfläche vorgesehen sein, beispielsweise deckungsgleich mit dem kreisförmigen Folienflecken 42 und exakt unter diesem angeordnet (in Figur 6c durch den Folienflecken 42 verdeckt). Selbstverständlich können auch andere Anzahlen, Geometrien oder Positionen in nahezu unbegrenzter Variationsmöglichkeit sowohl für den Folienflecken 42 als auch für einen eventuell auf der anderen Seiten liegenden Flecken zum Einsatz kommen.

Das vierte Ausführungsbeispiel 50 in Figur 6d trägt auf einem ersten wellenkopfförmigen Abschnitt 51 und einem zweiten wellenkopfförmigen Abschnitt 52 jeweils einen streifenförmigen Schließbereich 11 mit einem Hakenmaterial auf der Innenseite des Windelverschlussbandes. Insofern ist ein Überstandsabschnitt 53 auf den freien Überstand neben den Schließbereichen 11 begrenzt. Das Windelverschlussband des vierten Ausführungsbeispiels 50 hat zwei wellenkopfförmige Bereiche 15 am Benutzerende, auf welchen die Schließbereiche 11 jeweils identisch positioniert sind. Dementsprechend sind auch die Überstandsabschnitte 53 in Größe und Form identisch.

Auf den Überstandsabschnitten 53 sind eine erste Folie 54 bzw. eine zweite Folie 55 auflaminiert, welche an der Bandkante 16 mit dieser deckungsgleich liegen und an einem inneren Rand 56 parallel zu den Begrenzungen der jeweiligen Schließbereiche 11 geformt sind. Die beiden Folien 54, 55 sind jeweils etwa 600 µm dick und mit einer Prägung versehen (an der Folie 55 nicht dargestellt). Die Prägung der Folie 54 umfasst zahlreiche gerade und gekrümmte Linien, die jeweils zu mehreren miteinander gruppiert sind, im vorliegenden Beispiel ergeben sich hierdurch Buchstaben. Die einzelnen Gruppierungen, also im vorliegenden Beispiel die Buchstaben, sind in etwa geometrisch ähnlich zur Bandkante 16 verlaufend angeordnet, sodass sich durch einen ungeprägten Randbereich 57 der Folie 54 ein deutlich spürbarer Greifrand außerhalb der Buchstaben ergibt.

Wie insbesondere anhand Figur 1 ersichtlich, wird das Windelverschlussband nach vorliegender Erfindung vorzugsweise aus mehreren Schichten gebildet, die miteinander verbunden sind. So weist das beispielhaft aufgeführte Windelverschlussband nach Figur 1 eine Trägerfolie 80 auf, welche einerseits in einem Befestigungsbereich Klebestreifen 81 zur Befestigung an einem Releaseband 82 aufweist, wobei das Releaseband 82 seinerseits einen Klebestreifen 83 zur Befestigung einer Windel trägt. Andererseits trägt die Trägerfolie in einem Schließbereich 11 eine Zwischenträgerfolie 84 mittels eines Klebestreifens 85, welche wiederum über einen Klebestreifen 86 das Hakenmaterial 9 sowie das Bauelement 100 trägt. Wie unmittelbar ersichtlich ist somit das Bauelement 100 als separate Baugruppe ausgebildet und kann so, nachdem die Anordnung nach Figur 1 durch entsprechende Trennvorgänge zu an eine Windel applizierbaren Windelverschlussbändern ausgebildet ist, als separater Greifbereich 24 dienen. Der Vollständigkeit sei noch erwähnt, dass das Trägerband 80 in einem Zwischenbereich ein elastisches Band 87, beispielsweise aus TPE, tragen und im Bereich des Bandes geschlitzt sein kann. Hierdurch wird das Trägerband in seinem Zwischenbereich an sich elastisch streckbar. Es versteht sich, dass in einer alternativen Ausführungsform auch beispielsweise die Trägerfolie 80 und/oder die Zwischenträgerfolie 84 als Greifbereich ausgebildet werden kann, indem auf die separate Baugruppe 100 sowie den darunter liegenden Bereich des Klebestreifens 86, ggf. auch den darunter liegenden Bereich der Zwischenträgerfolie 84 sowie den entsprechenden Bereich des Klebestreifens 85, verzichtet und an dieser Stelle die Trägerfolie 80 und/oder die Zwischenträgerfolie 84 mit einer makroskopisch strukturierten Oberfläche versehen wird. Hierdurch wird auch ein separater Greifbereich geschaffen, da dieser strukturell aus einem anderen Material gebildet ist als die benachbarte funktionelle Baugruppe, nämlich der Schließbereich 11, welcher nach wie vor durch das die Haken aufweisende Hakenmaterial gebildet wird.

Nochmals sei darauf hingewiesen, dass sich die Erfindung nicht in den beispielhaft vorgestellten Ausführungsformen erschöpft.

## Patentansprüche

1. Verschlussband (10) für einen Hygieneartikel (8), insbesondere für eine Babywindel oder für eine Inkontinenzwindel, mit einem Befestigungsbereich zum permanenten Befestigen am Hygieneartikel (8) und mit einem Schließbereich (11) zum gleichzeitigen lösbaren Verbinden mit einer Oberfläche des Hygieneartikels (8), wobei das Verschlussband (10) einen Überstandsabschnitt (14) mit einem separaten Greifbereich (24) mit einer makroskopisch strukturierten Oberfläche (23, 33, 34) zwischen dem Schließbereich (11) und einem dem Schließbereich zuzuordnenden Bandende (16) aufweist, ***dadurch gekennzeichnet, dass*** die strukturierte Oberfläche eine Prägung aufweist.

2. Verschlussband nach Anspruch 1, ***dadurch gekennzeichnet, dass*** die Prägung eine Mehrzahl von geraden und/oder gekrümmten Linien aufweist, von denen vorzugsweise einige miteinander verbunden sind.

3. Verschlussband (10) für einen Hygieneartikel (8), insbesondere für eine Babywindel oder für eine Inkontinenzwindel, mit einem Befestigungsbereich zum permanenten Befestigen am Hygieneartikel (8) und mit einem Schließbereich (11) zum gleichzeitigen lösbaren Verbinden mit einer Oberfläche des Hygieneartikels (8), wobei das Verschlussband (10) einen Überstandsabschnitt (14) mit einem separaten Greifbereich (24) mit einer makroskopisch strukturierten Oberfläche (23, 33, 34) zwischen dem Schließbereich (11) und einem dem Schließbereich zuzuordnenden Bandende (16) aufweist, ***dadurch gekennzeichnet, dass*** der separate Greifbereich (24) ebenso wie der Schließbereich (11) auf einer Innenseite des Verschlussbands (10) angeordnet ist.

4. Verschlussband (10) für einen Hygieneartikel (8), insbesondere für eine Babywindel oder für eine Inkontinenzwindel, mit einem Befestigungsbereich zum permanenten Befestigen am Hygieneartikel (8) und mit einem Schließbereich (11) zum gleichzeitigen lösbaren Verbinden mit einer Oberfläche des Hygieneartikels (8), wobei das Verschlussband (10) einen Überstandsabschnitt (14) mit einem separaten Greifbereich (24) mit einer makroskopisch strukturierten Oberfläche (23, 33, 34) zwischen dem Schließbereich (11) und einem dem Schließbereich zuzuordnenden Bandende (16) aufweist, ***dadurch gekennzeichnet, dass*** der Greifbereich (24) streifenartig ausgebildet ist und in seiner Form zumindest teilweise im wesentlichem einem Greifrand (25) entsprechend bzw. einer Bandkante (25) entsprechend verläuft.

5. Verschlussband nach einem der vorhergehenden Ansprüche, ***dadurch gekennzeichnet, dass*** der Greifbereich an einem Greifrand des Überstandsabschnitts angeordnet ist.

6. Verschlussband nach einem der vorhergehenden Ansprüche 1 bis 4, ***dadurch gekennzeichnet, dass*** der Greifbereich mit einem Versatz zu einer Bandkante des Überstandsabschnitts angeordnet ist.

7. Verschlussband nach einem der vorhergehenden Ansprüche, ***dadurch gekennzeichnet, dass*** der Greifbereich einen etwa gleichen Abstand zu dem Schließbereich wie zu einer Bandkante aufweist.

8. Verschlussband nach einem der vorhergehenden Ansprüche, ***dadurch gekennzeichnet, dass*** der Greifbereich mäanderförmig verläuft.

9. Verschlussband nach einem der vorhergehenden Ansprüche, ***dadurch gekennzeichnet, dass*** beide Seiten des Verschlussbands einen gemeinsamen Greifbereich und/oder getrennte Greifbereiche aufweisen.

10. Verschlussband nach Anspruch 9, ***gekennzeichnet durch*** zwei sich deckende und gleich große Greifbereiche auf den beiden Seiten des Verschlussbands.

11. Verschlussband nach einem der vorhergehenden Ansprüche ***gekennzeichnet durch*** zwei separate folienartige Greifbereiche mit unterschiedlichen makroskopisch strukturierten Oberflächen.

12. Hygieneartikel, insbesondere Babywindel oder Inkontinenzwindel, mit einem Verschlussband nach einem der vorhergehenden Ansprüche.

13. Verfahren zum Herstellen eines Verschlussbands (10) nach einem der Ansprüche 1 bis 11, für einen Hygieneartikel (8), insbesondere für eine Babywindel oder für eine Inkontinenzwindel, ***dadurch gekennzeichnet, dass*** eine geprägte makroskopisch oberflächenstrukturierte (23, 33, 34) Folie (22; 31; 42; 54, 55; 100) von einer Rolle oder einer Spule auf einen Überstandsabschnitt des Verschlussbands laminiert wird.

14. Verfahren zum Herstellen eines Verschlussbands, nach einem der Ansprüche 1 bis 11, für einen Hygieneartikel, insbesondere für eine Babywindel oder für eine Inkontinenzwindel, ***dadurch gekennzeichnet, dass*** eine strukturell eigene Baugruppe, wie ein thermoplastisches Material, TPE (thermoplastische Elastomere) und/oder ein Heißsiegelklebstoff, auf einen Überstandsabschnitt des Verschlussbands aufgebracht wird und mit einer Prägewalze und/oder einem Prägerad geprägt wird.

## Claims

1. A closure tape (10) for a sanitary product (8), more specifically for a baby diaper or for an incontinence diaper, with a fastening region for permanent fixture to the sanitary product (8) and with a closing region (11) for concurrent releasable connection with a surface of the sanitary product (8), said closure tape (10) having a protruding portion (14) with a separate grip region (24) having a surface (23, 33, 34) with a macroscopic structure between said closing region (11) and a tape end (16) to be associated with said closing region, ***characterized in that*** said structured surface has an embossment.

2. The closure tape as set forth in claim 1, ***characterized in that*** the embossment comprises a plurality of straight and/or curved lines of which some are preferably connected together.

3. A closure tape (10) for a sanitary product (8), more specifically for a baby diaper or for an incontinence diaper, with a fastening region for permanent fixture to the sanitary product (8) and with a closing region (11) for concurrent releasable connection with a surface of the sanitary product (8), said closure tape (10) having a protruding portion (14) with a separate grip region (24) having a surface (23, 33, 34) with a macroscopic structure between the closing region (11) and a tape end (16) to be associated with said closing region, ***characterized in that*** said separate grip region (24) and said closure region (11) are both disposed on an inner side of said closure tape (10).

4. A closure tape (10) for a sanitary product (8), more specifically for a baby diaper or for an incontinence diaper, with a fastening region for permanent fixture to the sanitary product (8) and with a closing region (11) for concurrent releasable connection with a surface of the sanitary product (8), said closure tape (10) having a protruding portion (14) with a separate grip region (24) having a surface (23, 33, 34) with a macroscopic structure between the closing region (11) and a tape end (16) to be associated with said closing region, ***characterized in that*** said grip region (24) is formed like a tape extending at least in parts so as to substantially conform to a grip border (25) or to a tape edge (25).

5. The closure tape as set forth in any one of the afore mentioned claims, ***characterized in that*** the grip region is disposed at a grip border of the protruding portion.

6. The closure tape as set forth in any one of the claims 1 through 4, ***characterized in that*** the grip region is disposed offset with respect to a tape edge of the protruding portion.

7. The closure tape as set forth in any one of the afore mentioned claims, ***characterized in that*** the grip region is spaced approximately the same distance from the closure region and from a tape edge.

8. The closure tape as set forth in any one of the afore mentioned claims, ***characterized in that*** the grip region has a sinusoidal course.

9. The closure tape as set forth in any one of the afore mentioned claims, ***characterized in that*** both sides of the closure tape have one common grip region and/or separate grip regions.

10. The closure tape as set forth in claim 9, ***characterized by*** two coinciding grip regions of the same size provided on either side of said closure tape.

11. The closure tape as set forth in any one of the afore mentioned claims, ***characterized by*** two separate foil-like grip regions having surfaces exhibiting different macroscopic structures.

12. A sanitary product, more specifically a baby diaper or an incontinence diaper, with a closure tape as set forth in any one of the afore mentioned claims.

13. A method of manufacturing a closure tape (10) as set forth in any one of the claims 1 through 11, for a sanitary product (8), more specifically for a baby diaper or for an incontinence diaper, ***characterized in that*** an embossed foil (22; 31; 42; 54; 55; 100) having a macroscopically surface structure (23, 33, 34) is laminated from a roll or a bobbin onto a protruding portion of said closure tape.

14. A method of manufacturing a closure tape as set forth in any one of the claims 1 through 11, for a sanitary product, more specifically for a baby diaper or for an incontinence diaper, ***characterized in that*** a component having its own structure, such as a thermoplastic material, TPE (thermoplastic elastomers) and/or a heat seal adhesive is applied to a protruding portion of the closure tape and is embossed using an embossing roller and/or an embossing wheel.

## Revendications

1. Bande de fermeture (10) pour article hygiénique (8), notamment pour couche pour bébé ou couche incontinence, avec une zone de fixation pour la fixation permanente à l'article hygiénique (8) et avec une zone de fermeture (11) pour la liaison amovible simultanée à une surface de l'article hygiénique (8), la bande de fermeture (10) comportant, entre la zone de fermeture (11) et une extrémité de bande (16) destinée à être associée avec la zone de fermeture, une partie en débord (14) avec une zone de préhension (24) séparée dont une surface (23, 33, 34) présente une structure macroscopique, ***caractérisée en ce que*** la surface structurée comporte un gaufrage.

2. Bande de fermeture selon la revendication 1, ***caractérisée en ce que*** le gaufrage comporte une pluralité de lignes droites et/ou courbes dont certaines sont de préférence reliées entre elles.

3. Bande de fermeture (10) pour article hygiénique (8), notamment pour couche pour bébé ou couche incontinence, avec une zone de fixation pour la fixation permanente à l'article hygiénique (8) et avec une zone de fermeture (11) pour la liaison amovible simultanée à une surface de l'article hygiénique (8), la bande de fermeture (10) comportant, entre la zone de fermeture (11) et une extrémité de bande (16) destinée à être associée avec la zone de fermeture, une partie en débord (14) avec une zone de préhension (24) séparée dont une surface (23, 33, 34) présente une structure macroscopique, ***caractérisée en ce que*** la zone de préhension séparée (24) tout comme la zone de fermeture (11) sont disposées sur une face interne de la bande de fermeture (10).

4. Bande de fermeture (10) pour article hygiénique (8), notamment pour couche pour bébé ou couche incontinence, avec une zone de fixation pour la fixation permanente à l'article hygiénique (8) et avec une zone de fermeture (11) pour la liaison amovible simultanée à une surface de l'article hygiénique (8), la bande de fermeture (10) comportant, entre la zone de fermeture (11) et une extrémité de bande (16) destinée à être associée avec la zone de fermeture, une partie en débord (14) avec une zone de préhension (24) séparée dont une surface (23, 33, 34) présente une structure macroscopique, ***caractérisée en ce que*** la zone de préhension (24) est conformée en forme de bande s'étendant du moins en partie de manière à épouser un bord de préhension (25) ou une arête de bande (25).

5. Bande de fermeture selon l'une quelconque des revendications précédentes, ***caractérisée en ce que*** la bande de préhension est disposée sur un bord de préhension de la partie en débord.

6. Bande de fermeture selon l'une quelconque des revendications précédentes 1 à 4, ***caractérisée en ce que*** la zone de préhension est décalée par rapport à une arête de la bande formant la partie en débord.

7. Bande de fermeture selon l'une quelconque des revendications précédentes, ***caractérisée en ce que*** la zone de préhension se trouve à égale distance de la zone de fermeture et de l'arête de la bande.

8. Bande de fermeture selon l'une quelconque des revendications précédentes, ***caractérisée en ce que*** la zone de préhension fait des méandres.

9. Bande de fermeture selon l'une quelconque des revendications précédentes, ***caractérisée en ce que*** les deux faces de la bande de fermeture comportent une zone de préhension commune et/ou des zones de préhension séparées.

10. Bande de fermeture selon la revendication 9, ***caractérisée par*** deux zones de préhension de même taille qui coïncident sur les deux faces de la bande de fermeture.

11. Bande de fermeture selon l'une quelconque des revendications précédentes, ***caractérisée par*** deux zones de préhension en forme de feuille avec des surfaces présentant des structures macroscopiques différentes.

12. Article hygiénique, notamment couche pour bébé ou couche incontinence, avec une bande de fermeture selon l'une quelconque des revendications précédentes.

13. Procédé de réalisation d'une bande de fermeture (10) selon l'une quelconque des revendications 1 à 11, pour un article d'hygiène (8), notamment pour une couche pour bébé ou pour une couche incontinence, ***caractérisé en ce* qu'**une feuille (22 ; 31 ; 42 ; 54, 55 ; 100) gaufrée présentant une surface à structure macroscopique (23, 33, 34) est laminée à partir d'un rouleau ou d'une bobine sur une partie en débord de la bande de fermeture.

14. Procédé de réalisation d'une bande de fermeture selon l'une quelconque des revendications 1 à 11, pour un article d'hygiène, notamment pour une couche pour bébé ou pour une couche incontinence, ***caractérisé en ce qu'**un* composant possédant une structure propre, par exemple une matière thermoplastique, du TPE (élastomères thermoplastiques) et/ou un adhésif thermosoudable, est appliqué sur une partie en débord de la bande de fermeture et est gaufré avec un rouleau de gaufrage et/ou une roue de gaufrage.
